(19) Europäisches Patentamt
European Patent Office
Office européen des brevets

(11) **EP 4 449 982 B1**

(12) **EUROPEAN PATENT SPECIFICATION**

(45) Date of publication and mention
of the grant of the patent:
**28.05.2025 Bulletin 2025/22**

(51) International Patent Classification (IPC):
**A61B 5/11** (2006.01)　　**A61B 5/024** (2006.01)
**A61B 5/0245** (2006.01)　　A61B 5/00 (2006.01)

(21) Application number: **24198660.3**

(22) Date of filing: **06.10.2016**

(52) Cooperative Patent Classification (CPC):
**A61B 5/1121; A61B 5/02405; A61B 5/0245;
A61B 5/1102; A61B 5/363; A61B 5/6804;
A61B 5/686; A61B 5/6898; A61B 5/7257;
A61B 5/7282;** A61B 5/316; A61B 2562/0219

(54) **METHOD AND APPARATUS FOR PRODUCING INFORMATION INDICATIVE OF CARDIAC CONDITION**

VERFAHREN UND VORRICHTUNG ZUR ERZEUGUNG VON INFORMATIONEN ZUR ANZEIGE DES HERZZUSTANDS

PROCÉDÉ ET APPAREIL DE PRODUCTION D'INFORMATIONS INDIQUANT UN ÉTAT CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.10.2015 FI 20155703**

(43) Date of publication of application:
**23.10.2024 Bulletin 2024/43**

(62) Document number(s) of the earlier application(s) in accordance with Art. 76 EPC:
**16787911.3 / 3 359 037**

(73) Proprietor: **Precordior Oy**
**20520 Turku (FI)**

(72) Inventors:
• **AIRAKSINEN, Juhani**
**20300 Turku (FI)**
• **KOIVISTO, Tero**
**20320 Turku (FI)**
• **PÄNKÄÄLÄ, Mikko**
**21200 Raisio (FI)**
• **VALTONEN, Tuomas**
**20540 Turku (FI)**
• **JAFARI TADI, Mojtaba**
**20540 Turku (FI)**

(74) Representative: **Väänänen, Janne Kalervo**
**Vanarix Oy**
**Laaksolahdentie 74**
**02730 Espoo (FI)**

(56) References cited:
WO-A1-03/061473　　WO-A1-2013/160538
WO-A1-2015/036925　　US-A1- 2014 221 859

## Description

### Field of the disclosure

The disclosure relates generally to producing information indicative of cardiac condition. The produced information may indicate e.g. myocardial infarction. More particularly, the disclosure relates to an apparatus and to a method for producing information indicative of cardiac condition. Furthermore, the disclosure relates to a computer program for producing information indicative of cardiac condition.

### Background

Many abnormalities that may occur in the heart may lead to severe consequences if not diagnosed and appropriately treated and/or remedied. For example, myocardial infarction "MI" is one of the most serious cardiovascular diseases which happen due to obstruction of the coronary arteries. There are two types of MIs, STEMI, which refers to ST-elevation myocardial infarction, and NSTEMI, which refers to non-ST-elevation myocardial infarction. The names reflect differences in the electrocardiography "ECG" tracings. A STEMI heart attack is caused when a blood clot suddenly forms, completely blocking an artery in the heart. This can result in damage that covers a large area of the heart and extends deep into the heart muscle. NSTEMI heart attacks are different from STEMI heart attacks in several ways. With NSTEMI, the heart attack damage usually does not extend through the full depth of the heart muscle. The STEMI infarction may lead to a very dangerous situation if appropriate actions, such as e.g. balloon angioplasty, for opening the obstructed vessel are not carried out within a sufficiently short time from the occurrence of the myocardial infarction. An inherent problem related to the myocardial infarction as well as some other cardiac abnormalities is that the symptoms related to the myocardial infarction and the other cardiac abnormalities resemble many times the symptoms of plain heartburn. This may lead to situations where an individual, in the heart of which myocardial infarction or some other cardiac abnormality is developing, may erroneously think that there is plain heartburn and thus the individual under consideration does not recognise the seriousness of the situation at a sufficiently early stage. The consequences may be severe if the required treatment is too much delayed. On the other hand, the healthcare resources can be severely overloaded if for example a team for cardiac surgery is alerted and prepared in many cases which later turn out to be plain heartburn cases.

Currently, methods such as cardiography based on electromagnetic phenomena related to cardiac activity, echocardiography, and cardiography based on cardiovascular motion are used in the identification and assessment of various cardiac abnormalities. A well-known example of the cardiography based on electromagnetic phenomena related to cardiac activity is the electrocardiography "ECG", and examples of the cardiography based on cardiovascular motion are ballistocardiography "BCG" and seismocardiography "SCG". The echocardiography provides images of sections of the heart and can provide comprehensive information about the structure and function of the heart, but requires expensive equipment and specialised operating personnel. The ECG provides a fairly rapid electrical assessment of the heart, but does not provide any information relating to forces of contraction. The cardiography based on cardiovascular motion involves measurement of a signal indicative of cardiovascular motion. Earlier, the signal was obtained while an individual lay on a bed that was provided with an apparatus for measuring movements or there was a facilitating apparatus that was attached across the shin area of the legs. Currently, the signal can be obtained using small sensor elements, e.g. accelerometers, which are suitable for measuring minute movements which are representative of movements of the heart. The above-described methods are, however, not well suitable for an individual who is at home or elsewhere where specialised medical personnel are not present and who needs at least indicative information whether symptoms in the chest are caused by plain heartburn or by a more serious situation, e.g. cardiac ischemia such as myocardial infarction, ventricular tachycardia, or carditis such as myocarditis, pericarditis, perimyocarditis, or myopericarditis.

Publication WO03061473 describes a system for determining cardiac conditions based on a fast Fourier transform analysis of cardiac rotation signals. Publication EP2198916 describes an implantable telemetric device for measuring electromechanical parameters of the heart. The telemetric device comprises a sensor and corresponding processing means for detecting data relating to both the rotation of the heart and the mechanical vibrations which correspond to the first heart sound "FHS" and the second heart sound "SHS". The telemetric device further comprises means which use these data for diagnostic and/or therapeutic and/or monitoring purposes.

Publication US2007032749 describes a system for monitoring cardiac function in a human patient. The system comprises a motion sensor positioned at the heart and configured to sense movement of the apex of the heart. The system comprises a motion analysis element in operable communication with the motion sensor. The motion analysis element is configured to receive signals representative of the movement of the heart from the motion sensor and to process the received signals so that the signals are compared to at least one predetermined baseline value.

Publication WO2010145009 describes an apparatus for determining information indicative of a subject's physiological condition. The apparatus comprises: a) a sensor device configured to obtain ballistocardiograph data indicative of heart motion of the subject measured along a plurality of spatial axes, and b) a computing device communicatively coupled to the sensor device and configured to receive the ballistocardiograph data. The computing device is configured to determine, based on the ballistocardiograph data, processed data indicative of heart motion of the subject. The computing device is further configured to determine one or more indications of the subject's physiological condition based on the processed data.

Publication WO2013121431 describes a system for monitoring heart performance. The system comprises: (a) a motion sensor for sensing heart movement, (b) an electrical sensor for sensing an electrical activity of the heart, and (c) a processing unit for processing information received from the motion sensor and the electrical sensor.

The systems and devices described in the above-mentioned publications are, however, not well suitable for an individual who is at home or elsewhere where specialised medical personnel are not present and who needs at least indicative information whether symptoms in the chest are caused by plain heartburn or by a more serious situation, e.g. cardiac ischemia such as myocardial infarction, ventricular tachycardia, or carditis such as myocarditis, pericarditis, perimyocarditis, or myopericarditis.

## Summary

The following presents a simplified summary in order to provide basic understanding of some aspects of various invention embodiments. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying embodiments of the invention. The invention is defined by the appended claims.

In accordance with the invention, there is provided a new method for producing information indicative of cardiac conditions. A method according to the disclosure comprises:

- receiving a rotation signal indicative of rotational movement of a chest of an individual, the rotation signal being at least partly indicative of cardiac rotation,
- forming one or more indicator quantities each being derivable from an energy spectral density "ESD" based on one or more samples of the rotation signal each having a temporal length so that each sample has a non-zero temporal duration i.e. is a clip of the rotation signal, an average frequency corresponding to a center-of-mass of the ESD representing one of the one or more indicator quantities, and
- forming an indicator of cardiac condition on the basis of the one or more indicator quantities in accordance with a predetermined rule, the indicator of cardiac condition being indicative of the condition of the heart, i.e. whether the heart is in the normal state or in an abnormal state corresponding to for example cardiac ischemia such as myocardial infarction, ventricular tachycardia, or carditis such as myocarditis, pericarditis, perimyocarditis, or myopericarditis.

It has been noted that myocardial infarction "MI" as well as many other cardiac abnormalities cause changes in the above-mentioned energy spectral density which describes how the energy of the one or more samples of the rotation signal is distributed over different frequencies. Myocardial infarction and many other cardiac abnormalities change the myocardium twisting and untwisting performances and correspondingly ventricular muscles are not acting normally. Thus, with the aid of the one or more indicator quantities, it is possible to distinguish between for example myocardial infarction and heartburn.

The above-mentioned rotation signal can be produced for example with a gyroscope or another rotation sensor for obtaining a gyrocardiogram "GCG" i.e. a signal which is proportional to the rotation of the chest caused by the rotational movement of the heart. The rotation is measured advantageously with respect to three mutually orthogonal geometric axes typically called x-, y- and z-axes. In this case, the rotation signal has three components each of which has its own energy spectral density and can be used for determining the indicator of cardiac condition.

The heart rotation is a clinical parameter, i.e. a main cardiac feature, responsible for circa 60 % of the stroke volume generated by the heart. It has been noted that changes in indicator quantities which are derivable from the above-mentioned energy spectral density provide a warning for potential cardiac problems. An indicator quantity derivable from the above-mentioned energy spectral density can be for example the energy of a sample of the rotation signal. It is worth noting that, albeit the above-mentioned energy is derivable from the energy spectral density, the energy can as well be computed on the basis of the sample in the time-domain. Thus, for obtaining the energy, it is not necessary to compute the energy spectral density.

In conjunction with the invention, it has been noted that for example the STEMI infarction causes an increase in the above-mentioned energy related to the rotation of the chest. This differs from the results reported by E. Marcelli, L. Cercenelli, M. Musaico, P. Bagnoli, M.L. Costantino, R. Fumero, and G. Plicchi "Assessment of Cardiac Rotation by Means of Gyroscopic Sensors" Computers in Cardiology 2008;35:389-392, Università di Bologna, Bologna, Italy. The publication of E. Marcelli et. al. presents results obtained with a gyroscope which is directly on the surface of the heart. According to these results, an acute ischemia causes a significant decrease in both the measured maximum angle and the maximum value of angular velocity. Thus, the situation that for example the STEMI infarction causes an increase in the above-mentioned energy related to the rotation of the chest is not foreseeable from the results reported by E. Marcelli et. al.

An advantage of many gyroscopes is that the operation is not affected by gravity. Thus, the measurement is practically independent of the position or posture of the monitored individual. It has been noted that the external angular motion of the chest is orders of magnitude larger than what one could anticipate from the mere extent of the heart rotation and the ratio between the size of the heart and the diameter of the human chest. It has also been noted that the detection of the angular motion is also relatively insensitive to the posture of the sensor with respect to the heart. Thus, relatively accurate measurements can be made with even one gyroscope, for example microelectromechanical gyroscope, mechanically connected to the chest of an individual under consideration and configured to measure the rotation signal with respect to one geometric axis. Microelectromechanical gyroscopes are accurate, small in size, and commercially available.

It is also possible that a sensor element for measuring the rotation signal is an implantable element suitable for measuring the above-mentioned rotation signal when being placed under the skin of the chest of an individual under consideration.

In accordance with the invention, there is provided also a new apparatus for producing information indicative of cardiac condition. The apparatus according to the invention comprises a processing system for receiving a rotation signal indicative of rotational movement of a chest of an individual, the rotation signal being at least partly indicative of cardiac rotation. The processing system is configured to:

- form one or more indicator quantities each being derivable from an energy spectral density "ESD" based on one or more samples of the rotation signal each having a temporal length, an average frequency corresponding to a center-of-mass of the ESD representing one of the one or more indicator quantities, and

- form an indicator of cardiac condition on the basis of the one or more indicator quantities in accordance with a predetermined rule.

The apparatus may further comprise a sensor element, e.g. a gyroscope, for measuring the rotation signal. It is, however, emphasized that the apparatus does not necessarily comprise any sensor element but the apparatus may comprise a signal interface for connecting to an external sensor element.

The apparatus can be or can further comprise for example a mobile phone, a tablet computer, a piece of clothing that comprises the above-mentioned processing system and possibly also the sensor element i.e. wearable electronics, or another device which can be used by an individual who needs at least indicative information whether symptoms in his/her chest might implicate a severe situation. It is worth noting that in some cases the apparatus can be for example a single integrated circuit "IC" that is configured to constitute the above-mentioned processing system.

In accordance with the invention, there is provided also a new computer program for producing information indicative of cardiac condition. The computer program comprises computer executable instructions for controlling a programmable processing system to:

- form one or more indicator quantities each being derivable from an energy spectral density "ESD" based on one or more samples of a rotation signal indicative of rotational movement of a chest of an individual, the rotation signal being at least partly indicative of cardiac rotation and each of the one or more samples having a temporal length, and an average frequency corresponding to a center-of-mass of the ESD representing one of the one or more indicator quantities, and

- form an indicator of cardiac condition on the basis of the one or more indicator quantities in accordance with a predetermined rule.

In accordance with the invention, there is provided also a new computer program product. The computer program product comprises a non-volatile computer readable medium, e.g. a compact disc "CD", encoded with a computer program according to the invention.

Exemplifying and non-limiting embodiments of the invention are described in accompanied dependent claims.

Various exemplifying and non-limiting embodiments of the invention both as to constructions and to methods of operation, together with additional objects and advantages thereof, will be best understood from the following description of specific exemplifying embodiments when read in connection with the accompanying drawings.

The verbs "to comprise" and "to include" are used in this document as open limitations that neither exclude nor require the existence of also un-recited features. The features recited in the accompanied dependent claims are mutually freely combinable unless otherwise explicitly stated. Furthermore, it is to be understood that the use of "a" or "an", i.e. a singular form, throughout this document does not exclude a plurality.

**Brief description of figures**

Exemplifying and non-limiting embodiments of the invention and their advantages are explained in greater detail below with reference to the accompanying drawings, in which:

figure 1 shows a flowchart of a method according to an exemplifying and non-limiting embodiment of the invention for producing information indicative of cardiac condition,

figure 2 illustrates exemplifying energy spectral densities related to rotational movement of a chest with respect to three mutually orthogonal geometric axes in a normal case and in a case of the STEMI infraction, and

figure 3 shows a schematic illustration of an apparatus according to an exemplifying and non-limiting embodiment of the invention for producing information indicative of cardiac condition.

## Description of exemplifying and non-limiting embodiments

The specific examples provided in the description below should not be construed as limiting the scope and/or the applicability of the appended claims. Lists and groups of examples provided in the description are not exhaustive unless otherwise explicitly stated. The invention is defined by the appended claims.

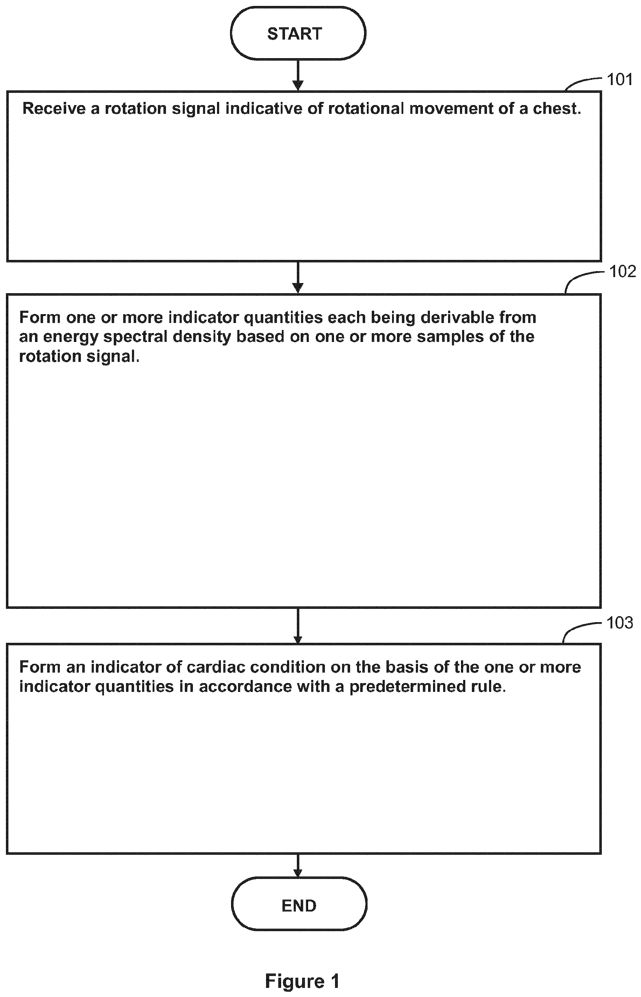

Figure 1 shows a flowchart of a method according to an exemplifying and non-limiting embodiment of the invention for producing information indicative of cardiac condition. The method comprises the following actions:

- action 101: receiving a rotation signal indicative of rotational movement of a chest of an individual, the rotation signal being at least partly indicative of cardiac rotation,
- action 102: forming one or more indicator quantities each being derivable from an energy spectral density "ESD" based on one or more samples of the rotation signal each having a temporal length, and
- action 103: forming an indicator of cardiac condition on the basis of the one or more indicator quantities in accordance with a predetermined rule.

The indicator of cardiac condition is capable of expressing cardiac abnormality and/or the risk of cardiac abnormality. The cardiac abnormality can be for example cardiac ischemia such as myocardial infarction, or carditis such as myocarditis, pericarditis, perimyocarditis, or myopericarditis.

The indicator of cardiac condition can be formed for example by comparing each indicator quantity to one or more threshold values. In cases where there is only one indicator quantity which is compared to only one threshold value, the indicator of cardiac condition is a yes/no-type two-valued data item. In cases where there are at least two indicator quantities and/or there are at least two threshold values for an indicator quantity, the indicator of cardiac condition can express different levels of the risk of cardiac abnormality. Threshold values related to a given indicator quantity may constitute a series of threshold values so that each threshold value represents a specific probability of myocardial infarction and/or some other cardiac abnormality.

Correspondingly, threshold values related to a many indicator quantities may constitute a series of threshold value groups each containing threshold values for the indicator quantities so that each threshold value group represents a specific probability of myocardial infarction and/or some other cardiac abnormality. Each threshold value can be determined on the basis of data gathered from a single person or a group of persons. A threshold value is not necessary constant but the threshold value can be changing according to the individual under consideration, according to time, and/or according to some other factors.

It is worth noting that the indicator of cardiac condition is not necessarily based on threshold values of the kind mentioned above. It is also possible that the indicator of cardiac condition is formed with a mathematical formula the input of which is/are the one or more indicator quantities and an output of which is the probability of cardiac abnormality.

The above-mentioned rotation signal can be produced for example with a gyroscope or another rotation sensor for measuring, from outside the chest, a signal which is proportional to the rotation of the chest caused by the rotational movement of the heart. It is also possible that the rotation signal is produced with an implant element placed under the skin of the chest.

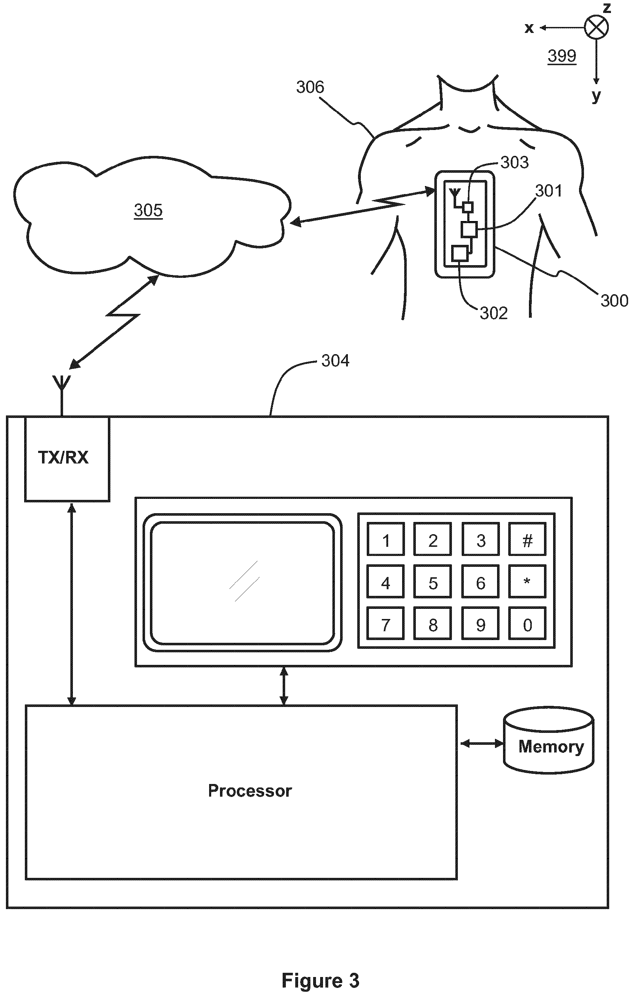

The rotation signal can be expressed as time-dependent angular speed with respect to a given geometric axis. In order to improve the reliability of the detection of cardiac condition, the rotation is measured advantageously with respect to three mutually orthogonal geometric axes typically called x-, y- and z-axes. The directions of the x-, y- and z-axes with respect to an individual's body are shown in figure 3. In this case, the rotation signal has three components each of which has its own energy spectral density.

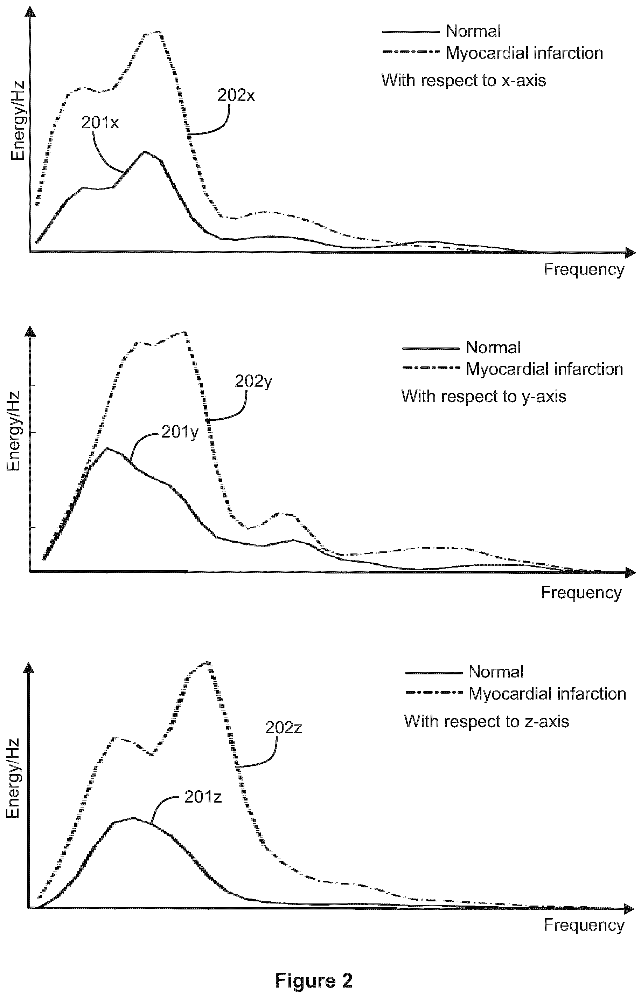

Figure 2 illustrates exemplifying energy spectral densities based on one or more samples of the rotational signal measured with respect to three mutually orthogonal geometric axes in a normal case and in a case of the STEMI infraction. The energy spectral densities related to the x-, y, and z-axes in the normal case are denoted with figure references 201x, 201y, and 201z. Correspondingly, the energy spectral densities related to the x-, y, and z-axes in the case of the STEMI infraction are denoted with figure references 202x, 202y, and 202z. For the normal case, it is possible to compute a combined energy spectral density so that the combined energy spectral density is the sum of the energy spectral densities 201x, 201y, and 201z. Correspondingly, for the STEMI infraction case, it is possible to compute a combined energy spectral density so that the combined energy spectral density is the sum of the energy spectral densities 202x, 202y, and 202z.

The exemplifying energy spectral densities shown in figure 2 are computed with the Welch method where a time domain signal under consideration is first split up into overlapping samples, the overlapping samples are then windowed with a suitable window function, a discrete Fourier transform is computed for each windowed sample, the results of the discrete Fourier transform are squared, and then the squared results are averaged so as to reduce the effect of noise. In this case, the temporal length of each sample is advantageously one heartbeat period or two or more successive heartbeat periods. The computed energy spectral density can be converted into a power spectral density "PSD" by dividing the values of the energy spectral density with the temporal length of the samples. The discrete Fourier transform can be e.g. the fast Fourier transform "FFT".

As can be seen from figure 2, the energy spectral densities 201x, 201y, and 201z in the normal case differ significantly from the corresponding energy spectral densities 202x, 202y, and 202z in the case of myocardial infraction.

A method according to an exemplifying and non-limiting embodiment of the invention comprises detecting one or more indications of acute myocardial infarction "AMI". Acute myocardial infarction is one of the most serious heart conditions and it should be detected with high accuracy as early as possible to allow clinical intervention. Measured data which is indicative of cardiac rotation and acceleration is advantageously divided into non-overlapping data segments each of which represents a respective one of non-overlapping and successive time periods. The temporal length of each time period can be e.g. 10 seconds or another suitable temporal length. The measured data can be preprocessed with e.g. Fast Fourier Transform "FFT" filtering and potential noise exclusion. Multiple features can be detected from the preprocessed data. The detected features may comprise for example one or more of the following: heartbeat rate variation, heartbeat rate, turning point ratio, spectral entropy, modifications of these, and/or Local Binary Patterns "LBPs" which describe the shape of a signal. There are multiple variants of LBPs such as for example Dominant LBPs and LBPs with Gabor filtering as a preprocessing step. In addition to the above-mentioned features, it is possible to use other features such as moments, spectrograms, wavelets, or Fourier Transform based features, e.g. Short-time Fourier Transform.

Each of the above-mentioned data segments can be divided into the following six components: Accelerometer X i.e. acceleration in the x-direction, Accelerometer Y, Accelerometer Z, Gyro X i.e. rotation around the x-axis, Gyro Y, and Gyro Z. For each of the x-, y-, and z-directions, a direction-specific acceleration feature vector "ACC_FV" and a direction-specific gyroscopic feature vector "GYRO_FV" can be extracted from the data segment components related to the direction under consideration. Thereafter, the resulting six feature vectors are combined for classification into a concatenated feature vector having the length of six times the length of each individual feature vector. If the individual feature vectors related to the x-, y-, and z-directions are: ACC_FV_x, ACC_FV_y, ACC_FV_z, GYRO_FV_x, GYRO_FV_y and GYRO_FV_z, the concatenated feature vector is: [ACC_FV_x, ACC_FV_y, ACC_FV_z, GYRO_FV_x, GYRO_FV_y, GYRO_FV_z].

In a method according to an exemplifying and non-limiting embodiment of the invention, a binary classifier using e.g. 10-fold cross validation is trained to classify data including at last a part of the concatenated feature vector into the following two classes: AMI and NON_AMI. Training data of the binary classifier can correspond to e.g. pre- and post-operation conditions of patients so that the training data comprises data portions corresponding to AMI situations and other data portions corresponding to situations without AMI. The classification can be performed with suitable machine learning classifiers including for example: Support Vector Machine "SVM", Kernel SVM "KSVM", and Random Forest "RF". There are also many other suitable classifiers such as Convolutional Neural Networks, Deep Convolutional Neural Networks, the Bayes Classifier, and other supervised or unsupervised classifiers. Unsupervised classification, such as clustering, can be used for example to aid the design of a supervised classifier. The classification results of the AMI detection can be reported via a user interface and/or recoded in a memory. It is also possible to use a classifier which is capable of detecting other classes than simply the above-mentioned AMI and NON_AMI. For example, the classifier can be trained to separate noise as the third class, or separate a totally different heart condition such as atrial fibrillation as additional class or any number N of heart abnormalities, i.e. N classes. Furthermore, the classes of the classifier do not need to be strict, i.e. the classifier may also return probabilities of the classes for further processing. Sometimes the length of the concatenated feature vector can be very long, and in these cases it may be advantageous to reduce the length with feature vector length reduction methods such as Principal Component Analysis "PCA", or Independent Component Analysis "ICA", or any other suitable method. This may increase the performance of the machine learning algorithm.

An apparatus according to an exemplifying and non-limiting embodiment of the invention comprises a processing system for receiving a rotation signal indicative of rotational movement of a chest and an acceleration signal indicative of acceleration of the chest, where the rotation and acceleration signals are at least partly indicative of cardiac rotation and acceleration. The processing system is configured to carry out the above-described method for detecting one or more indications of acute myocardial infarction "AMI".

An apparatus according to an exemplifying and non-limiting embodiment of the invention is a smartphone or another device which comprises an accelerometer and a gyroscope. In order to detect indications of AMI, one can place the smartphone on the chest of a patient when the patient is in supine position. Then, a measurement recording is taken from the patient. The procedure is non-invasive and can be carried out without support from medical staff and/or other similar persons. An apparatus according to an exemplifying and non-limiting embodiment of the invention is a wearable device or an implantable device. An apparatus according to an exemplifying and non-limiting embodiment of the invention is configured to send information about a patient to a remote place, such as a hospital first aid clinic or equivalent, or simply inform the user about his/her condition. The accelerometer and the gyroscope can be for example microelectromechanical systems "MEMS".

A method according to an exemplifying and non-limiting embodiment of the invention comprises detecting one or more indications of Ventricular and/or Atrial Tachycardia. Tachycardia is a rapid increase in the resting heart rate so that individual's heart rate irregularly, i.e. without any physical activity or external stress, exceeds the normal rate, e.g. >100 beat per minute. Generally, abnormal electrical impulses in the ventricle or atrium result in a rapid heart rate and control the individual's heart pumping action rhythm. A method according to an exemplifying and non-limiting embodiment of the invention is based on the hypothesis that gyroscopic signals indicate different conditions of the heart such as tachycardia, both atrial and ventricular tachycardias, so that by considering several features of the gyroscopic signals it is feasible to classify tachycardia from normal rhythm. Considering the tachycardia features, it is also possible to discriminate atrial tachycardia from ventricular tachycardia that is a very dangerous condition, possibly leading to sudden death. The following presents a simplified summary for detecting ventricular and atrial tachycardia using a gyroscope. The method is based on measuring the cardiac rotation and considering one or more features and/or indicator quantities which are related directly or indirectly to the energy/power spectral density of gyrocardiograms. It has been perceived that tachycardia as well as myocardial infarction "MI" causes changes in the total, i.e. absolute, rotation of the myocardium. Tachycardia impacts on myocardial twisting and untwisting performances and correspondingly ventricular and atrial muscles are not performing normally. With the aid of suitable features and/or indicator quantities, one is able to distinguish tachycardia from normal rhythm. Furthermore, one can distinguish atrial tachycardia from ventricular tachycardia.

The method for detecting one or more indications of Ventricular and/or Atrial Tachycardia is based on the perception that, in general tachycardia conditions, heart rate and peak-to-peak amplitude of the rotational signals, i.e. GCG, suddenly increase and exceed the normal level. More precisely, the heart rate rapidly increases to more than 100 beats per minute "bpm" in the resting condition which is the primary sign to diagnose tachycardia. Similarly, the amplitude gets typically at least two times greater than in the normal rhythm. This change in the amplitude of the rotation signal is a unique feature to recognize tachycardia episodes in the signal.

After detecting a tachycardia episode, it is critical to diagnose the type of tachycardia. The beat-to-beat intervals in ventricular tachycardia "VT" have been noted varying irregularly meaning that VT causes considerable heartbeat rate variation "HRV", i.e. time deviation between consecutive heartbeat periods, whereas, in atrial tachycardia there is no or negligible heartbeat rate variation. Thus, estimating the HRV indexes can be used to classify between ventricular and atrial tachycardia. It has been also noted that the total or absolute cardiac rotation in tachycardia situations significantly differs from that of the normal rhythm. Considering the normal rhythm rotations as a baseline, ventricular tachycardia applies less myocardial rotation, twisting + untwisting, while the atrial tachycardia enforces more rotation to myocardium. Therefore, those episodes where the cardiac rotation is less than the above-mentioned baseline can be considered ventricular tachycardia and those with greater rotational values can be considered atrial tachycardia. Therefore, changes in the total myocardial rotation can be taken into the account since rotational information can be treated as the indicator of cardiac condition.

Indicators can be considered in the frequency domain as well. As mentioned above, the heartbeat rate variation is significant during ventricular tachycardia and thus no single prominent frequency component can be found on the energy/power spectral density of the gyroscopic signals while the ventricular tachycardia is present. For another example, using the autocorrelation technique one can simply determine whether there is a significant frequency component in the gyroscopic signal or not. Lack of prominent side peaks in an autocorrelation plot can be treated as an indicator of the ventricular tachycardia, while prominent side peaks in the autocorrelation plot means that the considered episode could be atrial or sinus tachycardia. The above-described approach can be used for indicating tachycardia. Furthermore, the above-described approach can be used for indicating the type of detected tachycardia, i.e. ventricular or atrial tachycardia. Several different time and frequency domain features and indicators can be described for identification of the cardiac condition.

An apparatus according to an exemplifying and non-limiting embodiment of the invention comprises a processing system for receiving a rotation signal indicative of rotational movement of a chest of an individual, where the rotation signal is at least partly indicative of cardiac rotation. The processing system is configured to carry out the above-described method for detecting one or more indications of Ventricular and/or Atrial Tachycardia. An apparatus according to an exemplifying and non-limiting embodiment of the invention is a smartphone or another suitable device which comprises a gyroscope.

At least one indicator quantity is defined to be the average frequency corresponding to the center-of-mass of the energy spectral density ESD(f). An increase in the average frequency is indicative of an increased risk of cardiac abnormality, e.g. cardiac ischemia such as myocardial infarction, or carditis such as myocarditis, pericarditis, perimyocarditis, or myopericarditis. The average frequency can be computed according to the following equation:

$$f_{av} = \frac{\int_B f\ ESD\ (f)\ df}{\int_B ESD\ (f)\ df},$$

where $f_{av}$ is the above-mentioned average frequency corresponding to the center-of-mass of the energy spectral density.

As mentioned above, the indicator of cardiac condition is formed on the basis of the one or more indicator quantities in accordance with the predetermined rule. A method according to an exemplifying and non-limiting embodiment of the invention comprises determining one or more parameters of the predetermined rule on the basis of one or more samples of a rotation signal measured from an individual when the individual is in the normal state. The one or more parameters of the predetermined rule can be for example one or more threshold values which are compared to the one or more indicator quantities. In this case, the method comprises forming one or more normal-state indicator quantities corresponding to the normal case and determining the one or more threshold values on the basis of the one or more normal-state indicator quantities. Each threshold value can be for example a value that is a predetermined percentage, e.g. 25%, greater than the corresponding normal-state indicator quantity.

A method according to an exemplifying and non-limiting embodiment of the invention comprises detecting the length of a time interval from the AO-peak caused by an opening of the aortic valve to the AC-peak caused by a subsequent closure of the aortic valve, and forming the indicator of cardiac condition on the basis of the one or more indicator quantities and the detected length of the AO-AC time interval. The length of the AO-AC time interval can be detected for example from the above-mentioned rotation signal and/or from another signal related to movements of the heart. The other signal can be produced with for example a 1-, 2-, or 3-axis acceleration sensor.

The detected length of the AO-AC time interval can be used for improving the reliability of the detection of cardiac abnormalities. In a normal state, the length of the AO-AC time interval is typically about 30% of the heartbeat period. During myocardial infarction, the length of the AO-AC time interval increases typically up to about 50% of the heartbeat period. During panic disorder, the energy related to twisting of the hearth may increase compared to the normal state, but the length of the AO-AC time interval does not increase in the same way as during myocardial infarction. Thus, the length of the AO-AC time interval can be used for distinguishing between myocardial infarction and panic disorder.

A method according to an exemplifying and non-limiting embodiment of the invention comprises detecting, from a signal related to operation of a heart, a heartbeat rate and forming the indicator of cardiac condition on the basis of the above-mentioned one or more indicator quantities and the detected heartbeat rate. The heartbeat rate can be detected for example from the above-mentioned rotation signal and/or from another signal related to movements of the heart and/or an electrocardiography "ECG" signal. The other signal related to movements of the heart can be produced with for example a 1-, 2-, or 3-axis acceleration sensor.

A high heartbeat rate, typically > 100 beats/minute and even up to 300 beats/minute, together with an increase in the energy/amplitude related to the rotation signal is indicative of ventricular tachycardia which may have very severe consequences if not appropriately treated and/or remedied.

A method according to an exemplifying and non-limiting embodiment of the invention comprises:

- 　receiving an electrocardiography "ECG" signal,

- 　detecting the length of a time interval between the R-peak of the electrocardiography signal and the highest peak of the rotation signal corresponding to a same heartbeat period - advantageously this is done for many heartbeat periods so as to improve reliability, and

- 　forming the indicator of cardiac condition on the basis of the above-mentioned one or more indicator quantities, the detected heartbeat rate, and the detected length of the time interval.

An increase in the detected length of the time interval is a factor indicative of ventricular tachycardia, and thus the detected length of the time interval can be used for improving the reliability of the detection of ventricular tachycardia.

A method according to an exemplifying and non-limiting embodiment of the invention comprises hemodynamical measurements such as systolic time intervals (STI) and diastolic time intervals (DTI). A method according to an exemplifying and non-limiting embodiment of the invention comprises detecting particular mechanical cardiac events, for example, instants of mitral valve closure (MC), aortic valve opening (AO), mitral valve opening (MO) and aortic valve closure (AC). Systolic time intervals (STI) including total electromechanical systole (QS2), left ventricular ejection time (LVET)/ AO-AC time interval and pre-ejection period (PEP) can be measured for example by combining an electrocardiography (ECG) signal for investigating cardiac condition. The QS2 is measured from the Q-wave of the QRS complex in ECG to the point of the aortic closure (AC) in the rotational signal. In addition to QS2, PEP and LVET are two important indexes to assess the cardiac contractility and correspondingly cardiac condition. PEP is the time interval from the Q-wave to the onset of AO peak, the first high amplitude component of the rotation signal. LVET is the time interval between the moments of aortic valve opening and closing in the cardiac cycle. A huge change in the normal value of the rotational STIs, i.e. PEP and LVET, together with increase in the energy/amplitude related to the rotational signal is indicative of the cardiac abnormality such as heart arrhythmias.

A method according to an exemplifying and non-limiting embodiment of the invention comprises optionally measuring the rotation signal with a sensor element from an individual's body. A method according to another exemplifying and non-limiting embodiment of the invention comprises reading the rotation signal from a memory, in which case the rotation signal has been measured earlier and recorded to the memory. A method according to an exemplifying and non-limiting embodiment of the invention comprises receiving the rotation signal from an external data transfer system. Therefore, the measuring is not an essential and necessary step of methods according to many embodiments of the invention, but the rotation signal is to be understood as an input quantity of the methods.

A computer program according to an exemplifying and non-limiting embodiment of the invention comprises computer executable instructions for controlling a programmable processing system to carry out actions related to a method according to any of the above-described exemplifying embodiments of the invention.

A computer program according to an exemplifying and non-limiting embodiment of the invention comprises software modules for producing information indicative of cardiac condition. The software modules comprise computer executable instructions for controlling a programmable processing system to:

-    form one or more indicator quantities each being derivable from an energy spectral density based on one or more samples of a rotation signal indicative of rotational movement of a chest, the rotation signal being at least partly indicative of cardiac rotation and each of the one or more samples having a temporal length, and

-    form an indicator of cardiac condition on the basis of the one or more indicator quantities in accordance with a predetermined rule.

The software modules can be e.g. subroutines or functions implemented with a suitable programming language and with a compiler suitable for the programming language and for the programmable processing system under consideration. It is worth noting that also a source code corresponding to a suitable programming language represents the computer executable software modules because the source code contains the information needed for controlling the programmable processing system to carry out the above-presented actions and compiling changes only the format of the information. Furthermore, it is also possible that the programmable processing system is provided with an interpreter so that a source code implemented with a suitable programming language does not need to be compiled prior to running.

A computer program product according to an exemplifying and non-limiting embodiment of the invention comprises a computer readable medium, e.g. a compact disc "CD", encoded with a computer program according to an embodiment of invention.

A signal according to an exemplifying and non-limiting embodiment of the invention is encoded to carry information defining a computer program according to an embodiment of invention. The signal can be used for configuring e.g. a mobile phone, a tablet computer, wearable electronics, or another device which comprises an appropriate processing system and a sensor element for measuring the rotation signal. The signal, i.e. the computer program, can be delivered to the mobile phone, tablet computer, wearable electronics, or other device in many different ways. For example, the signal, i.e. the computer program, can be downloaded from the Internet. For example, cloud services or the like can be utilized for the delivery of the signal, i.e. the computer program, and/or for delivery of detection results to medical personnel.

Figure 3 shows a schematic illustration of an apparatus 300 according to an exemplifying and non-limiting embodiment of the invention for producing information indicative of cardiac condition. The apparatus comprises a processing system 301 for receiving a rotation signal indicative of rotational movement of a chest of an individual, the rotation signal being at least partly indicative of cardiac rotation. The processing system 301 is configured to form one or more indicator quantities each being derivable from an energy spectral density based on one or more samples of the rotation signal, where each sample has a temporal length that can be one or more heart-beat periods. The processing system 301 is configured to form an indicator of cardiac condition on the basis of the one or more indicator quantities in accordance with a predetermined rule. The processing system 301 is advantageously configured to control a display of the apparatus 300 to show the indicator of cardiac condition.

The processing system 301 can be implemented with one or more processor circuits, each of which can be a programmable processor circuit provided with appropriate software, a dedicated hardware processor such as for example an application specific integrated circuit "ASIC", or a configurable hardware processor such as for example a field programmable gate array "FPGA".

In the exemplifying case illustrated in figure 3, the apparatus 300 comprises a sensor element 302 for measuring the above-mentioned rotation signal. The sensor element is communicatively connected to the processing system 301. The sensor element 302 is suitable for measuring the rotation signal when being outside the chest and in a direct or indirect mechanical contact with the chest. The sensor element 302 can be for example a gyroscope or another rotation sensor for measuring a signal which is proportional to the rotation of the chest caused by the rotational movement of the heart. The gyroscope can be for example a miniature gyroscopic sensor of the piezoelectric fork type.

An apparatus according to another exemplifying and non-limiting embodiment of the invention does not comprise a sensor element but a corded or cordless signal interface for connecting to an external sensor element. In this case, the sensor element can be for example an implantable element suitable for measuring the above-mentioned rotation signal when being placed under the skin of the chest of an individual under consideration.

In the exemplifying case illustrated in figure 3, the apparatus 300 comprises a radio transmitter 303 and the processing system 301 is configured to control the radio transmitter to transmit an alarm signal in response to a situation in which the indicator of cardiac condition is indicative of cardiac abnormality. The apparatus 300 can be for example a mobile phone, a tablet computer, a piece of clothing, or another portable device. In the exemplifying case illustrated in figure 3, the apparatus 300 is configured to transmit the alarm signal to a monitoring system 304 via a data communication network 305, e.g. a cellular network. The monitoring system 304 can be located for example in a hospital or in other premises where specialised medical personnel are present. In addition to the alarm signal, the apparatus 300 can be configured to transmit the measured rotation signal to the monitoring system 304 so as to enable the monitoring system and the medical personnel to analyse the rotation signal. In addition to the sensor element 302 for measuring the rotation signal, the apparatus 300 may further comprise one or more other sensor elements for measuring other information from the individual 306. The processing system 301 can be configured to control the radio transmitter 303 to transmit the other information to the monitoring system 304. The one or more other sensor elements may comprise for example a three-axis accelerometer which is capable of measuring translational movements independently in three mutually orthogonal directions x, y, and z of e.g. the coordinate system 399 shown in figure 3.

It is to be noted that in cases where the apparatus 300 transmits the rotation signal to the monitoring system 304, the functionality for forming the one or more indicator quantities and for forming the indicator of cardiac condition can be implemented also in the monitoring system 304, or only in the monitoring system 304. In this case, the monitoring system 304 is actually an apparatus according to an embodiment of the invention. Thus, apparatuses according to different embodiments of the invention can be constructed in various ways.

The processing system 301 is configured to compute the energy spectral density and to compute the average frequency corresponding to the center-of-mass of the energy spectral density. The average frequency represents an indicator quantity and is defined according to the following:

$$f_{av} = \frac{\int_B f\ ESD\ (f)\ df}{\int_B ESD\ (f)\ df},$$

where $f_{av}$ is the average frequency. The processing system 301 is configured to set the indicator of cardiac condition to express cardiac abnormality when the average frequency $f_{av}$ exceeds a threshold value.

As mentioned above, the processing system 301 is configured to form the indicator of cardiac condition on the basis of the one or more indicator quantities in accordance with the predetermined rule. In an apparatus according to an exemplifying and non-limiting embodiment of the invention, the processing system 301 is configured to determine one or more parameters of the predetermined rule on the basis of the one or more indicator quantities in response to reception of a user control signal from a user interface of the apparatus. The one or more parameters of the predetermined rule can be for example one or more threshold values that are compared to the one or more the indicator quantities. With the aid of the user control signal, the individual 306 can train the apparatus so that the one or more parameters of the predetermined rule, e.g. one or more threshold values, are tuned to be suitable for the individual 306. The training is carried out by controlling the apparatus 300 to determine the one or more parameters on the basis of such value or values of the one or more indicator quantities which correspond to the normal state of the individual 306. For example, a threshold value can be a value that is a predetermined percentage, e.g. 25%, greater than the corresponding indicator quantity related to the normal state. For another example, the above-mentioned predetermined rule can be a mathematical formula and one or more parameters of the mathematical formula can be adjusted so that the probability of cardiac abnormality given by the mathematical formula is zero when one or more indicator quantities which correspond to the normal state are inputted to the mathematical formula.

In an apparatus according to an exemplifying and non-limiting embodiment of the invention, the processing system 301 is configured to detect, from the rotation signal and/or another signal related to movements of the heart, the length of a time interval from the AO-peak caused by an opening of the aortic valve to the AC-peak caused by a subsequent closure of the aortic valve, and to form the indicator of cardiac condition on the basis of the one or more indicator quantities and the detected length of the time interval.

In an apparatus according to an exemplifying and non-limiting embodiment of the invention, the indicator of cardiac condition is an indicator of cardiac ischemia.

In an apparatus according to an exemplifying and non-limiting embodiment of the invention, the indicator of cardiac condition is an indicator of myocardial infarction.

In an apparatus according to an exemplifying and non-limiting embodiment of the invention, the indicator of cardiac condition is an indicator of carditis.

In an apparatus according to an exemplifying and non-limiting embodiment of the invention, the indicator of cardiac condition is an indicator of myocarditis.

In an apparatus according to an exemplifying and non-limiting embodiment of the invention, the indicator of cardiac condition is an indicator of pericarditis.

In an apparatus according to an exemplifying and non-limiting embodiment of the invention, the indicator of cardiac condition is an indicator of perimyocarditis.

In an apparatus according to an exemplifying and non-limiting embodiment of the invention, the indicator of cardiac condition is an indicator of myopericarditis.

In an apparatus according to an exemplifying and non-limiting embodiment of the invention, the processing system 301 is configured to detect, from a suitable signal related to the operation of a heart, the heartbeat rate and to form the indicator of cardiac condition on the basis of the above-mentioned one or more indicator quantities and the detected heartbeat rate. **In** this case, the indicator of cardiac condition is an indicator of ventricular tachycardia.

In an apparatus according to an exemplifying and non-limiting embodiment of the invention, the processing system 301 is configured to:

- receive an electrocardiography "ECG" signal,

- detect the length of a time interval between the R-peak of the electrocardiography signal and the highest peak of the rotation signal corresponding to a same heartbeat period, and

- form the indicator of ventricular tachycardia on the basis of the one or more indicator quantities, the detected heartbeat rate, and the detected length of the time interval.

The specific examples provided in the description given above should not be construed as limiting the scope and/or the applicability of the appended claims. Lists and groups of examples provided in the description given above are not exhaustive unless otherwise explicitly stated.

**Claims**

1. An apparatus (300) comprising:

   - a processing system (301) for receiving a rotation signal indicative of rotational movement of a chest of an individual, the rotation signal being at least partly indicative of cardiac rotation,

   wherein the processing system is configured to:

   - form one or more indicator quantities each being derivable from an energy spectral density based on one or more samples of the rotation signal each having a temporal length, and
   - form an indicator of cardiac condition on the basis of the one or more indicator quantities in accordance with a predetermined rule.

   **characterized in that** the processing system is configured to compute the energy spectral density and to compute an average frequency corresponding to a center-of-mass of the energy spectral density, the average frequency representing one of the indicator quantities and being defined according to the following:

   $$f_{av} = \frac{\int_B f \; ESD(f) \; df}{\int_B ESD(f) \; df},$$

   where $f_{av}$ is the average frequency, ESD(f) is the energy spectral density, B is a frequency area of the energy spectral density, and f is frequency.

2. An apparatus according to claim 1, wherein the processing system is configured to detect, from a signal related to movements of a heart, a length of a time interval from an AO-peak caused by an opening of an aortic valve to an AC-peak caused by a subsequent closure of the aortic valve, and to form the indicator of cardiac condition on the basis of the one or more indicator quantities and the detected length of the time interval.

3. An apparatus according to claim 1 or 2, wherein the apparatus comprises a sensor element (302) for measuring the rotation signal, the sensor element being communicatively connected to the processing system.

4. An apparatus according to any one of claims 1-3, wherein the indicator of cardiac condition is an indicator of cardiac ischemia.

5. An apparatus according to claim 4, wherein the indicator of cardiac condition is an indicator of myocardial infarction.

6. An apparatus according to any one of claims 1-5, wherein the indicator of cardiac condition is an indicator of carditis.

7. An apparatus according to claim 6, wherein the indicator of cardiac condition is an indicator of at least one of the following: myocarditis, pericarditis, perimyocarditis, myopericarditis.

8. An apparatus according to any one of claims 1-7, wherein the processing system is configured to detect, from a signal related to operation of a heart, a heartbeat rate and to form the indicator of cardiac condition on the basis of the one or more indicator quantities and the detected heartbeat rate.

9. An apparatus according to claim 9, wherein the processing system is configured to:

   - receive an electrocardiography signal,
   - detect a length of a time interval from an R-peak of the electrocardiography signal to the highest peak of the rotation signal corresponding to a same heartbeat period, and
   - form the indicator of cardiac condition on the basis of the one or more indicator quantities, the detected heartbeat rate, and the detected length of the time interval.

10. An apparatus according to claim 8 or 9, wherein the indicator of cardiac condition is an indicator of tachycardia.

11. An apparatus according to claim 10, wherein the processing system is configured to detect a heartbeat rate variation from the detected heartbeat rate and to form the indicator of cardiac condition on the basis of the detected heartbeat rate variation, the indicator of cardiac condition being indicative of whether the tachycardia is ventricular tachycardia or atrial tachycardia.

12. A computer-implemented method comprising:

   - receiving (101), from a memory, a rotation signal recorded in the memory and indicative of rotational movement of a chest of an individual, the rotation signal being at least partly indicative of cardiac rotation,

wherein the method further comprises:

   - forming (102) one or more indicator quantities each being derivable from an energy spectral density based on one or more samples of the rotation signal each having a temporal length, and
   - forming (103) an indicator of cardiac condition on the basis of the one or more indicator quantities in accordance with a predetermined rule,

   **characterized in that** the method comprises computing the energy spectral density and computing an average frequency corresponding to a center-of-mass of the energy spectral density, the average frequency representing one of the indicator quantities and being defined according to the following:

$$f_{av} = \frac{\int_B f \; ESD\,(f) \; df}{\int_B ESD\,(f) \; df},$$

   where $f_{av}$ is the average frequency, $ESD(f)$ is the energy spectral density, B is a frequency area of the energy spectral density, and f is frequency.

13. A computer program comprising computer executable instructions for controlling a programmable processing system to:

   - receive a rotation signal indicative of rotational movement of a chest of an individual, the rotation signal being at least partly indicative of cardiac rotation,
   - form one or more indicator quantities each being derivable from an energy spectral density based on one or more samples of the rotation signal indicative of the rotational movement of the chest of an individual, the rotation signal being at least partly indicative of cardiac rotation and each of the one or more samples having a temporal length, and
   - form an indicator of cardiac condition on the basis of the one or more indicator quantities in accordance with a predetermined rule,

   **characterized in that** the computer program comprises computer executable instructions for controlling a programmable processing system to compute the energy spectral density and an average frequency corresponding to a center-of-mass of the energy spectral density, the average frequency representing one of the indicator quantities and being defined according to the following:

$$f_{av} = \frac{\int_B f\ ESD\ (f)\ df}{\int_B ESD\ (f)\ df},$$

where $f_{av}$ is the average frequency, ESD(f) is the energy spectral density, B is a frequency area of the energy spectral density, and f is frequency.

**Patentansprüche**

1. Vorrichtung (300), die Folgendes umfasst:

   - ein Verarbeitungssystem (301) zum Empfangen eines Rotationssignals, das auf eine Rotationsbewegung eines Brustkorbs eines Individuums schließen lässt, wobei das Rotationssignal zumindest teilweise auf eine Herzrotation schließen lässt,

   wobei das Verarbeitungssystem zu Folgendem konfiguriert ist:

   - eine oder mehrere Indikatorgrößen zu bilden, die jeweils von einer spektralen Energiedichte auf Grundlage einer oder mehrerer Proben des Rotationssignals, die eine zeitliche Länge aufweisen, ableitbar sind, und
   - einen Indikator eines Herzzustandes auf der Grundlage der einen oder der mehreren Indikatorgrößen in Übereinstimmung mit einer vorbestimmten Regel zu bilden,

   **dadurch gekennzeichnet, dass** das Verarbeitungssystem dafür konfiguriert ist, die spektrale Energiedichte zu berechnen und eine durchschnittliche Frequenz, die einem Massenmittelpunkt der spektralen Energiedichte entspricht, zu berechnen, wobei die durchschnittliche Frequenz eine der Indikatorgrößen darstellt und entsprechend dem Folgenden definiert ist:

   $$f_{av} = \frac{\int_B f\ ESD\ (f)\ df}{\int_B ESD\ (f)df},$$

   wobei $f_{av}$ die durchschnittliche Frequenz ist, ESD(f) die spektrale Energiedichte ist, B ein Frequenzbereich der spektralen Energiedichte ist und f eine Frequenz ist.

2. Vorrichtung nach Anspruch 1, wobei das Verarbeitungssystem dafür konfiguriert ist, aus einem Signal, das mit Bewegungen eines Herzes verknüpft ist, eine Länge eines Zeitabschnitts von einer AO-Spitze, die durch ein Öffnen einer Aortenklappe verursacht wird, bis zu einer AC-Spitze, die durch ein darauffolgendes Schließen der Aortenklappe verursacht wird, zu erfassen und den Indikator des Herzzustandes auf der Grundlage der einen oder der mehreren Indikatorgrößen und der erfassten Länge des Zeitabschnitts zu bilden.

3. Vorrichtung nach Anspruch 1 oder 2, wobei die Vorrichtung ein Sensorelement (302) zum Messen des Rotationsignals umfasst, wobei das Sensorelement kommunikativ mit dem Verarbeitungssystem verbunden ist.

4. Vorrichtung nach einem der Ansprüche 1 bis 3, wobei der Indikator eines Herzzustandes ein Indikator einer kardialen Ischämie ist.

5. Vorrichtung nach Anspruch 4, wobei der Indikator eines Herzzustandes ein Indikator eines Myokardinfarkts ist.

6. Vorrichtung nach einem der Ansprüche 1 bis 5, wobei der Indikator eines Herzzustandes ein Indikator einer Karditis ist.

7. Vorrichtung nach Anspruch 6, wobei der Indikator eines Herzzustandes ein Indikator von mindestens einem von Folgendem ist: Myokarditis, Perikarditis, Perimyokarditis, Myoperikarditis.

8. Vorrichtung nach einem der Ansprüche 1 bis 7, wobei das Verarbeitungssystem dafür konfiguriert ist, aus einen Signal, das mit einer Funktionsweise eines Herzes verknüpft ist, eine Herzschlagfrequenz zu erfassen und den Indikator eines Herzzustandes auf Grundlage der einen oder der mehreren Indikatorgrößen und der erfassten Herzschlagfrequenz zu bilden.

9. Vorrichtung nach Anspruch 8, wobei das Verarbeitungssystem zu Folgendem konfiguriert ist:

   - ein Elektrokardiographie-Signal zu empfangen,
   - eine Länge eines Zeitabstandes von einer R-Spitze des Elektrokardiographie-Signals bis zu der höchsten Spitze des Rotationssignals, das einem selben Herzschlagzeitraum entspricht, zu erfassen und
   - den Indikator eines Herzzustandes auf Grundlage der einen oder der mehreren Indikatorgrößen, der erfassten Herzschlagfrequenz und der erfassten Länge des Zeitabstandes zu bilden.

10. Vorrichtung nach Anspruch 8 oder 9, wobei der Indikator eines Herzzustandes ein Indikator einer Tachykardie ist.

11. Vorrichtung nach Anspruch 10, wobei das Verarbeitungssystem dafür konfiguriert ist, eine Herzschlagfrequenz-Abweichung von der erfassten Herzschlagfrequenz zu erfassen und den Indikator eines Herzzustandes auf Grundlage der erfassten Herzschlagfrequenz-Abweichung zu bilden, wobei der Indikator eines Herzzustandes darauf schließen lässt, ob die Tachykardie eine ventrikuläre Tachykardie oder eine atriale Tachykardie ist.

12. Rechnerimplementiertes Verfahren, das Folgendes umfasst:

- Empfangen (101), aus einem Speicher, eines Rotationssignals, das in dem Speicher aufgezeichnet ist und auf eine Rotationsbewegung eines Brustkorbs eines Individuums schließen lässt, wobei das Rotationssignal zumindest teilweise auf eine Herzrotation schließen lässt,

wobei das Verfahren ferner Folgendes umfasst:

- Bilden (102) einer oder mehrerer Indikatorgrößen, die jeweils von einer spektralen Energiedichte auf Grundlage einer oder mehrerer Proben des Rotationssignals, die eine zeitliche Länge aufweisen, ableitbar sind, und
- Bilden (103) eines Indikators eines Herzzustandes auf der Grundlage der einen oder der mehreren Indikatorgrößen in Übereinstimmung mit einer vorbestimmten Regel,

**dadurch gekennzeichnet, dass** das Verfahren das Berechnen der spektralen Energiedichte und das Berechnen einer durchschnittlichen Frequenz, die einem Massenmittelpunkt der spektralen Energiedichte entspricht, umfasst, wobei die durchschnittliche Frequenz eine der Indikatorgrößen darstellt und entsprechend dem Folgenden definiert ist:

$$f_{av} = \frac{\int_B f\ ESD\ (f)\ df}{\int_B\ ESD\ (f)df},$$

wobei $f_{av}$ die durchschnittliche Frequenz ist, ESD(f) die spektrale Energiedichte ist, B ein Frequenzbereich der spektralen Energiedichte ist und f eine Frequenz ist.

13. Rechnerprogramm, das rechnerausführbare Anweisungen zum Steuern eines programmierbaren Verarbeitungssystems zu Folgendem umfasst:

- ein Rotationssignal zu empfangen, das auf eine Rotationsbewegung eines Brustkorbs eines Individuums schließen lässt, wobei das Rotationssignal zumindest teilweise auf eine Herzrotation schließen lässt,
- eine oder mehrere Indikatorgrößen zu bilden, die jeweils von einer spektralen Energiedichte auf Grundlage einer oder mehrerer Proben des Rotationssignals, die eine zeitliche Länge aufweisen, ableitbar sind, und
- einen Indikator eines Herzzustandes auf der Grundlage der einen oder der mehreren Indikatorgrößen in Übereinstimmung mit einer vorbestimmten Regel zu bilden,

**dadurch gekennzeichnet, dass** das Rechnerprogramm rechnerausführbare Anweisungen zum Steuern eines programmierbaren Verarbeitungssystems umfasst, um die spektrale Energiedichte zu berechnen und eine durchschnittliche Frequenz zu berechnen, die einem Massenmittelpunkt der spektralen Energiedichte entspricht, wobei die durchschnittliche Frequenz eine der Indikatorgrößen darstellt und entsprechend dem Folgenden definiert ist:

$$f_{av} = \frac{\int_B f\ ESD\ (f)\ df}{\int_B\ ESD\ (f)df},$$

wobei $f_{av}$ die durchschnittliche Frequenz ist, ESD(f) die spektrale Energiedichte ist, B ein Frequenzbereich der spektralen Energiedichte ist und f eine Frequenz ist.

**Revendications**

1. Appareil (300) comprenant :

- un système de traitement (301) pour recevoir un signal de rotation indiquant un mouvement de rotation d'une poitrine d'un individu, le signal de rotation indiquant au moins partiellement une rotation cardiaque,

dans lequel le système de traitement est configuré pour :

- former une ou des quantité(s) indicatrice(s) pouvant être chacune dérivée d'une densité spectrale d'énergie en se basant sur un ou des échantillon(s) du signal de rotation ayant chacun une longueur temporelle, et
- former un indicateur d'une pathologie cardiaque en se basant sur la ou les quantité(s) indicatrice(s) conformément à une règle prédéterminée,

**caractérisé en ce que** le système de traitement est configuré pour calculer la densité spectrale d'énergie et pour calculer une fréquence moyenne correspondant à un centre de gravité de la densité spectrale d'énergie, la fréquence moyenne représentant une des quantités indicatrices et étant définie selon ce qui suit :

$$f_{av} = \frac{\int_B f \, ESD\,(f)\, df}{\int_B ESD\,(f) df},$$

où $f_{av}$ est la fréquence moyenne, ESD(f) est la densité spectrale d'énergie, B est une aire de fréquence de la densité spectrale d'énergie et f est la fréquence.

2. Appareil selon la revendication 1, dans lequel le système de traitement est configuré pour détecter, à partir d'un signal lié à des mouvements d'un cœur, une longueur d'un intervalle de temps d'un pic AO provoqué par une ouverture d'une valve aortique à un pic AC provoqué par une fermeture consécutive de la valve aortique, et pour former l'indicateur de pathologie cardiaque en se basant sur la ou les quantité(s) indicatrice(s) et la longueur détectée de l'intervalle de temps.

3. Appareil selon la revendication 1 ou 2, dans lequel l'appareil comprend un élément de capteur (302) pour mesurer le signal de rotation, l'élément de capteur étant connecté en communication au système de traitement.

4. Appareil selon l'une quelconque des revendications 1-3, dans lequel l'indicateur de pathologie cardiaque est un indicateur d'ischémie cardiaque.

5. Appareil selon la revendication 4, dans lequel l'indicateur de pathologie cardiaque est un indicateur d'infarctus du myocarde.

6. Appareil selon l'une quelconque des revendications 1-5, dans lequel l'indicateur de pathologie cardiaque est un indicateur de cardite.

7. Appareil selon la revendication 6, dans lequel l'indicateur de pathologie cardiaque est un indicateur d'au moins un de ce qui suit : myocardite, péricardite, périmyocardite, myopericardite.

8. Appareil selon l'une quelconque des revendications 1-7, dans lequel le système de traitement est configuré pour détecter, à partir d'un signal lié au fonctionnement d'un cœur, une fréquence cardiaque et pour former l'indicateur de pathologie cardiaque en se basant sur la ou les quantité(s) indicatrice(s) et la fréquence cardiaque détectée.

9. Appareil selon la revendication 9, dans lequel le système de traitement est configuré pour :

   - recevoir un signal d'électrocardiographie,
   - détecter une longueur d'un intervalle de temps d'un pic R du signal d'électrocardiographie au pic le plus élevé du signal de rotation correspondant à une même période de fréquence cardiaque, et
   - former l'indicateur de pathologie cardiaque en se basant sur la ou les quantité(s) indicatrice(s), la fréquence cardiaque détectée et la longueur détectée de l'intervalle de temps.

10. Appareil selon la revendication 8 ou 9, dans lequel l'indicateur de pathologie cardiaque est un indicateur de tachycardie.

11. Appareil selon la revendication 10, dans lequel le système de traitement est configuré pour détecter une variation de fréquence cardiaque à partir de la fréquence cardiaque détectée et pour former l'indicateur de pathologie cardiaque en se basant sur la variation de fréquence cardiaque détectée, l'indicateur de pathologie cardiaque indiquant si la tachycardie est une tachycardie ventriculaire ou une tachycardie atriale.

12. Procédé implémenté sur ordinateur comprenant :

   - de recevoir (101), d'une mémoire, un signal de rotation enregistré dans la mémoire et indiquant un mouvement de rotation d'une poitrine d'un individu, le signal de rotation indiquant au moins partiellement une rotation cardiaque,

   dans lequel le procédé comprend en outre :

   - de former (102) une ou des quantité(s) indicatrice(s) pouvant être chacune dérivée d'une densité spectrale d'énergie en se basant sur un ou des échantillon(s) du signal de rotation ayant chacun une longueur temporelle, et
   - de former (103) un indicateur d'une pathologie cardiaque en se basant sur la ou les quantité(s) indicatrice(s) conformément à une règle prédéterminée,

   **caractérisé en ce que** le procédé comprend de calculer la densité spectrale d'énergie et de calculer une fréquence moyenne correspondant à un centre de gravité de la densité spectrale d'énergie, la fréquence moyenne représentant une des quantités indicatrices et étant définie selon ce qui suit :

$$f_{av} = \frac{\int_B f \, ESD\,(f)\, df}{\int_B ESD\,(f) df},$$

où $f_{av}$ est la fréquence moyenne, ESD(f) est la densité spectrale d'énergie, B est une aire de fréquence de la densité spectrale d'énergie et f est la fréquence.

**13.** Programme informatique comprenant des instructions pouvant être exécutées sur ordinateur pour commander un système de traitement programmable à :

    - recevoir un signal de rotation indiquant un mouvement de rotation d'une poitrine d'un individu, le signal de rotation indiquant au moins partiellement une rotation cardiaque,
    - former une ou des quantité(s) indicatrice(s) pouvant être chacune dérivée d'une densité spectrale d'énergie en se basant sur un ou des échantillon(s) du signal de rotation indiquant le mouvement de rotation de la poitrine d'un individu, le signal de rotation indiquant au moins partiellement une rotation cardiaque et chacun du ou des échantillon(s) ayant une longueur temporelle, et
    - former un indicateur d'une pathologie cardiaque en se basant sur la ou les quantité(s) indicatrice(s) conformément à une règle prédéterminée,

**caractérisé en ce que** le programme informatique comprend des instructions pouvant être exécutées sur ordinateur pour commander un système de traitement programmable à calculer la densité spectrale d'énergie et une fréquence moyenne correspondant à un centre de gravité de la densité spectrale d'énergie, la fréquence moyenne représentant une des quantités indicatrices et étant définie selon ce qui suit :

$$f_{av} = \frac{\int_B f\ ESD\ (f)\ df}{\int_B ESD\ (f) df},$$

où $f_{av}$ est la fréquence moyenne, ESD(f) est la densité spectrale d'énergie, B est une aire de fréquence de la densité spectrale d'énergie et f est la fréquence.

START

Receive a rotation signal indicative of rotational movement of a chest.

— 101

Form one or more indicator quantities each being derivable from an energy spectral density based on one or more samples of the rotation signal.

— 102

Form an indicator of cardiac condition on the basis of the one or more indicator quantities in accordance with a predetermined rule.

— 103

END

Figure 1

Figure 2

**Figure 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03061473 A
- EP 2198916 A
- US 2007032749 A
- WO 2010145009 A
- WO 2013121431 A

**Non-patent literature cited in the description**

- **E. MARCELLI** ; **L. CERCENELLI** ; **M. MUSAICO** ; **P. BAGNOLI** ; **M.L. COSTANTINO** ; **R. FUMERO** ; **G. PLICCHI**. Assessment of Cardiac Rotation by Means of Gyroscopic Sensors. *Computers in Cardiology*, 2008, vol. 35, 389-392